# EUROPÄISCHE PATENTANMELDUNG

(11) **EP 4 403 143 A1**
(43) Veröffentlichungstag der Anmeldung: **24.07.2024**
(21) Anmeldenummer: 23152867.0
(22) Anmeldetag: 23.01.2023
(51) Int. Cl.: A61F 2/64, A61F 2/78, A61F 2/50

(54) **KNIEADAPTER ZUR VERBINDUNG EINER ENDOPROTHESE MIT EINER EXOPROTHESE**

(71) Anmelder: Back to Mobility GmbH, 50354 Hürth (DE)
(72) Erfinder: WÜNSCH, Benjamin, 50354 Hürth (DE); FISCHER, Maximilian, 50354 Hürth (DE)
(74) Vertreter: Grünecker Patent- und Rechtsanwälte PartG mbB

(57) **Zusammenfassung**

Die Erfindung betrifft einen Knieadapter (1) zur Verbindung einer Endoprothese (2) mit einer Exoprothese (3), wobei ein Kugelgelenk (4), wobei das Kugelgelenk (4) einen teilweise kugelförmigen Gelenkkopf (10) und eine dazu komplementär geformte Gelenkpfanne (5) aufweist, wobei der Gelenkkopf (10) in der Gelenkpfanne (5) gelagert ist, wobei der Gelenkkopf (10) dazu ausgebildet ist, an einem von Endo- und Exoprothese (2,3) angeordnet zu werden, wobei die Gelenkpfanne (5) dazu ausgebildet ist, an der anderen von Endo- und Exoprothese (2,3) angeordnet zu werden, wobei der Gelenkkopf (10) durch zumindest ein Scherelement (11) in der Gelenkpfanne (5) festgelegt ist.

## Beschreibung

Die vorliegende Erfindung betrifft einen Knieadapter zur Verbindung eines osseointegrierten Implantatsystems in Form einer Endoprothese mit einer Exoprothese. Der Knieadapter dient dazu, die Endoprothese vor hohen mechanischen Belastungen zu schützen und eine durch eine Unfallsituation verursachte Skelettfraktur zu vermeiden.

Jene überproportionalen Belastungen auf die Endoprothese können beispielsweise bei einer Stolperbewegung oder auch bei einem Sturz entstehen. Wirkt durch eine solche Unfallsituation eine überproportionale mechanische Belastung auf beispielsweise den Fußersatz, wird diese Belastung aufgrund der Längserstreckung der Beinprothese in Form eines starken Moments unmittelbar auf den Anbindungsbereich der in dem Knochen installierten Endoprothese weitergegeben. Resultierend aus dem langen Hebel und des dadurch wirkenden Moments auf die Osseointegration der Endoprothese, kann dies zu der Skelettfraktur führen.

Aus dem Stand der Technik sind Knieadapter bekannt, deren Funktion darin besteht, eben diese hohen Belastungen auf die in den Knochen integrierte Endoprothese zu begrenzen.

So offenbart die US 2015/0257904 A1 unter anderem einen Knieadapter zum Verbinden einer im Knochen verankerten Endoprothese mit einer Exoprothese. Der Knieadapter umfasst ein Hauptgehäuse mit einem ersten Befestigungsabschnitt, der zur Befestigung an der Endoprothese angeordnet ist, und einen zweiten Befestigungsabschnitt, der zur Befestigung an der Exoprothese angeordnet ist. Der Knieadapter umfasst insbesondere einen Sicherheitsmechanismus zum Schutz der im Knochen befestigten Endoprothese vor hohen mechanischen Kräften, einschließlich Rotations- und Biegekräften. Dazu umfasst der Sicherheitsmechanismus einen Mechanismus zur Auslösung bei Überschreitung einer zu hohen Rotationskraft. Ist der Sicherheitsmechanismus ausgelöst, lässt sich die Exoprothese um ihre axiale Erstreckung verdrehen. Ferner umfasst der Sicherheitsmechanismus einen Biegekraft-Auslösemechanismus. Wird ein vorbestimmtes Maß einer Querkraft überschritten, löst sich der Biegekraft-Auslösemechanismus und die Exoprothese kann in Kraftwirkungsrichtung verschwenken.

Nachteilig bei dem in der US 2015/0257904 A1 vorgeschlagenen Knieadapter ist jedoch, dass der Sicherheitsmechanismus beziehungsweise der Biegekraft-Auslösemechanismus nur bei überhöhten Querkräften auslöst, die entgegen einer Geradeausbewegung des Prothesenträgers, also orthogonal zu der Frontalebene wirken. Seitliche Querkräfte, also Kräfte die orthogonal zu der Sagittalebene wirken, oder Querkräfte von hinten, also ebenfalls orthogonal zu der Frontalebene, aber mit inverser Orientierung, führen nicht zu einem Auslösen des Sicherheitsmechanismus. Folglich wird ein entsprechendes Moment über die Exoprothese und den nicht auslösenden Knieadapter an die im Knochen angebundene Endoprothese weitergegeben.

Hiervon ausgehend liegt der vorliegenden Erfindung die Aufgabe zugrunde, die aus dem Stand der Technik bekannten Nachteile zumindest teilweise auszuräumen. Darüber hinaus soll ein benutzerfreundlicher und konstruktiv einfach gestalteter Knieadapter geschaffen werden.

Die Aufgabe wird gelöst mit einem Knieadapter mit den Merkmalen des unabhängigen Anspruchs 1. Weitere vorteilhafte Ausgestaltungen der Erfindung sind in den abhängig formulierten Ansprüchen angegeben. Die in den abhängig formulierten Ansprüchen einzeln aufgeführten Merkmale sind in technologisch sinnvoller Weise miteinander kombinierbar und können weitere Ausgestaltungen der Erfindung definieren. Darüber hinaus werden die in den Ansprüchen angegebenen Merkmale in der Beschreibung näher präzisiert und erläutert, wobei weitere bevorzugte Ausgestaltungen der Erfindung dargestellt werden.

Der erfindungsgemäße Knieadapter zur Verbindung einer Endoprothese mit einer Exoprothese umfasst ein Kugelgelenk, wobei das Kugelgelenk einen teilweise kugelförmigen Gelenkkopf und eine dazu komplementär geformte Gelenkpfanne aufweist, wobei der Gelenkkopf in der Gelenkpfanne gelagert ist, wobei der Gelenkkopf dazu ausgebildet ist, an einem von Endo- und Exoprothese angeordnet zu werden, wobei die Gelenkpfanne dazu ausgebildet ist, an der anderen von Endo- und Exoprothese angeordnet zu werden, wobei der Gelenkkopf durch zumindest ein Scherelement in der Gelenkpfanne festgelegt ist.

Vorsorglich sei angemerkt, dass die im Folgenden verwendeten Zahlenwörter ("erste", "zweite", ...) vorrangig (nur) zur Unterscheidung von mehreren gleichartigen Gegenständen, Größen oder Prozessen dienen, also insbesondere keine Abhängigkeit und/oder Reihenfolge dieser Gegenstände, Größen oder Prozesse zueinander zwingend vorgeben. Sollte eine Abhängigkeit und/oder Reihenfolge erforderlich sein, ist dies hier explizit angegeben oder es ergibt sich offensichtlich für den Fachmann beim Studium der konkret beschriebenen Ausgestaltung.

Der Knieadapter ist dazu ausgebildet, eine als zumindest teilweisen Oberschenkelersatz ausgebildete und osseointegrierte Endoprothese mit einer den Unterschenkel ersetzenden Exoprothese zu verbinden. Die Endoprothese (griech. endo "innen") ist ein künstliches Implantat, das im Rahmen einer Operation dauerhaft zumindest abschnittsweise in den Körper eingebracht wird. Es ersetzt krankhafte oder zerstörte Körperstrukturen. Mit einem zumindest teilweisen Oberschenkelersatz ist gemeint, dass die Endoprothese in einen Rest des Oberschenkelknochens, beispielsweise mit einem Ende in den restlichen Schaft des Oberschenkelknochens integriert ist, wobei das andere Ende aus dem Beinstumpf austritt.

Darüber hinaus ist der hier vorgeschlagene Knieadapter auch für eine Endoprothese geeignet, die den Oberschenkelknochen vollständig ersetzen kann.

Eine Prothese die vollständig außerhalb des Körpers angeordnet ist, bezeichnet man als Exoprothese (griech. exo "außen"). Diese kann beispielsweise mit einem Arm- oder Beinstumpf extern verbunden werden, wobei auch wie in diesem Fall, die Verbindung mit einer Endoprothese möglich ist.

Der Knieadapter umfasst ein Kugelgelenk, wobei das Kugelgelenk einen teilweise kugelförmigen Gelenkkopf und eine dazu komplementär geformte Gelenkpfanne aufweist. Die Gelenkpfanne und der Gelenkkopf sind so zueinander ausgebildet, dass der Gelenkkopf verschwenkbar innerhalb der Gelenkpfanne gelagert ist. Um den Gelenkkopf innerhalb der Gelenkpfanne festzulegen, ist zumindest ein Scherelement vorgesehen, das die Gelenkpfanne mit dem Gelenkkopf verbindet.

Vorzugsweise ist ein weiteres Scherelement vorgesehen, wobei das Scherelement und das weitere Scherelement orthogonal zueinander ausgerichtet sind, wobei das Scherelement und das weitere Scherelement jeweils mit einem Ende in der Gelenkpfanne und mit dem jeweils anderen Ende in dem Gelenkkopf angeordnet sind. Ferner bevorzugt können die Scherelemente auch in verschiedenen Winkelpositionen zueinander ausgerichtet sein, wobei insbesondere bevorzugt auch noch weitere Scherelemente vorgesehen sein können.

Um eine optimale Festlegung des Gelenkkopfes innerhalb der Gelenkpfanne zu ermöglichen, mit anderen Worten also eine Bewegung des Gelenkkopfes innerhalb der Gelenkpfanne zu sperren, sind das Scherelement und das weitere Scherelement vorgesehen. Die beiden Scherelemente sind jeweils mit einem Ende in der Gelenkpfanne und mit dem jeweils anderen Ende in dem Gelenkkopf angeordnet. Das Scherelement blockiert eine translatorische und rotatorische Bewegung des Gelenkkopfes innerhalb der Gelenkpfanne in eine Frontalebene und in eine Sagittalebene, wohingegen das weitere Scherelement eine translatorische und rotatorische Bewegung des Gelenkkopfes innerhalb der Gelenkpfanne in eine Transversalebene und in die Frontalebene blockiert. Das erste Scherelement blockiert in der Regel alle Rotationen bis auf eine Rotation um die proximale/laterale Achse, das zweite Element blockiert in der Regel alle Rotationen bis auf eine Rotation um die mediale/laterale Achse. Grundsätzlich ist aber auch eine inverse oder dazu gänzlich abweichende Anordnung der Scherelemente alternativ bevorzugt.

Als Frontalebene wird in der Anatomie eine Ebene bezeichnet, die sich beim aufrechtstehenden Menschen von oben nach unten wie von links nach rechts erstreckt. Eine Frontalebene teilt einen Körper in einen vorderen und einen hinteren Anteil. Als Sagittalebene wird in der Anatomie eine Ebene bezeichnet, die sich von oben nach unten wie von hinten nach vorne erstreckt. Eine Sagittalebene teilt einen Körper in einen rechten und einen linken Anteil. Als Transversalebene wird in der Anatomie eine Ebene bezeichnet, die im rechten Winkel zur Längsachse quer liegt. Sie spannt sich bei lotrechter Längsachse horizontal aus und erstreckt sich beim aufrechtstehenden Menschen von vorne nach hinten wie von links nach rechts. Eine solche Transversalebene wird auch Horizontalebene genannt und teilt einen Körper in einen oberen und einen unteren Anteil.

Die Scherelemente sind jeweils mit einer Sollbruchstelle ausgebildet. Wird ein bestimmtes Maß an Kraft oder Moment das auf sie wirkt überschritten, brechen sie und geben den Gelenkkopf innerhalb der Gelenkpfanne frei. Anschließend kann der Gelenkkopf innerhalb der Gelenkpfanne bevorzugt bis zu einem seitlichen Anschlag der Gelenkpfanne frei verschwenken, ohne eine Kraft auf die Verbindung zwischen Endoprothese und Knochen zu übertragen. Darüber hinaus wird die Exoprothese gegenüber der Endoprothese üblicherweise auch in rotatorischer Richtung freigegeben. Mit anderen Worten kann sich die Exoprothese gegenüber der Endoprothese bevorzugt auch in axialer Richtung verdrehen.

Der Vorteil des Kugelgelenks besteht darin, dass nach dem Bruch der Scherelemente die Exoprothese Querkräften oder Torsionsbelastungen in jegliche Richtung ausweichen kann und in deren Wirkungsrichtung verschwenkt. Somit ist lediglich ein Sicherheitsmechanismus in Form des Kugelgelenks nötig, um eine Absicherung gegenüber zu hohen mechanischen Belastungen herzustellen, die aus 360° in axialer Richtung auf die Exoprothese wirken könnten.

Vorzugsweise greift das Scherelement, ausgehend von der Innenseite des Gelenkkopfes, in die Gelenkpfanne ein, wobei das weitere Scherelement von der Außenseite der Gelenkpfanne in den Gelenkkopf eingreift. Die Anordnung des Scherelements innerhalb des Gelenkkopfes ermöglicht eine kompakte Bauweise des Knieadapters. Allerdings ist auch eine inverse Anordnung des jeweiligen Scherelements alternativ bevorzugt.

Vorzugsweise sind das Scherelement und das weitere Scherelement aus Edelstahl ausgebildet, wobei das Scherelement stärker dimensioniert ist als das weitere Scherelement und eine höhere Bruchfestigkeit gegenüber einer Scherbeanspruchung aufweist. Die Scherelemente sind jeweils aus einem spröden Edelstahl ausgebildet und weisen eine niedrige Duktilität auf. Eine niedrige Duktilität hat den Vorteil, dass bei einer genau festgelegten Belastung durch ein Moment oder eine Kraft, die als Scherkräfte auf das Scherelement wirken, das Scherelement bricht, also ohne eine vorhergehende plastische Verformung nachgibt. Da erfahrungsgemäß und nach Auswertungen die höchsten Belastungen in und entgegen der Fortbewegungsrichtung, also orthogonal zu der Frontalebene, auf die Exoprothese wirken, ist das Scherelement stärker dimensioniert als das weitere Scherelement. Belastungen quer zur Fortbewegungsrichtung, also orthogonal zu der Sagittalebene, des Nutzers fallen hingegen schwächer aus, weswegen das weitere Scherelement schwächer dimensioniert ist.

Vorzugsweise weist der Gelenkkopf auf der von der Gelenkpfanne abgewandten Seite ein Brückenmodul auf, wobei das Brückenmodul komplementär zu einem Verbindungsbereich der Endoprothese oder/und der Exoprothese ausgebildet ist. Die Endoprothese oder Exoprothese ist ihrerseits mit einem zu dem Brückenmodul komplementär ausgebildeten Verbindungsbereich versehen, der in das Brückenmodul aufnehmbar ist. Vorzugsweise ist das Brückenmodul als Hohlzylinder ausgebildet und einteilig mit dem Gelenkkopf verbunden. Das brückenmodulseitige Ende der Endoprothese oder Exoprothese ist hingegen komplementär zu der Innenseite des Hohlzylinders als Zylinder ausgebildet und kann in den Hohlzylinder eingeführt werden. Allerdings sind auch andere komplementäre Verbindungsformen zwischen dem Brückenmodul und der Endoprothese denkbar und mitunter vorteilhaft. Vorzugsweise kann sich der Hohlzylinder auch verjüngen beziehungsweise der zylindrische Abschnitt der Endoprothese in die von dem Brückenmodul abgewandte Richtung leicht aufweiten, um eine Presspassung beim Einführen herzustellen.

Zur kraftschlüssigen Verbindung der Endoprothese oder Exoprothese an dem Brückenmodul ist vorzugsweise an der der Gelenkpfanne abgewandten Seite des Brückenmoduls umfangsseitig ein Befestigungselement angeordnet. Bei dem Befestigungselement kann es sich um jegliche Einrichtung handeln, die eine kraft- und/oder reibschlüssige und/oder formschlüssige Verbindung zwischen dem Brückenmodul und der Endoprothese oder Exoprothese herstellt. Für die erleichterte Anwendbarkeit des Nutzers ist das Befestigungselement bevorzugt beispielsweise als ringförmiger Schnellspanner umlaufend um den Bereich des Brückenmoduls ausgebildet. Mithin wird ein Kraftschluss durch das Spannen eines Hebels bewirkt. Ferner bevorzugt ist auch eine formschlüssige Verbindung zwischen der Endoprothese und dem Brückenmodul durch das Befestigungselement.

Vorzugsweise weist der Gelenkkopf einen Äquator auf einer Normalenebene zu seiner axialen Erstreckung auf, wobei die Gelenkpfanne den Gelenkkopf über diesen Äquator hinweg in Richtung des Brückenmoduls zumindest teilweise umschließt. Durch diese Einnestung des Gelenkkopfes, bei Betrachtung des Knieadapters in axialer Richtung über die 90° hinweg, wird ein Herausfallen des Gelenkkopfes aus der Gelenkpfanne verhindert. Mit anderen Worten ist die Pfannenfläche der Gelenkpfanne mehr als halbkugelförmig ausgebildet, so dass sich ihr Radius im Öffnungsbereich bereits gegenüber dem Radius des Äquators verringert hat.

Vorzugsweise ist ein den Gelenkkopf radial umschließender Bereich der Gelenkpfanne in zumindest zwei Abschnitte unterteilt, wobei die Abschnitte durch Aussparungen in axialer Erstreckung der Gelenkpfanne, ausgehend von der dem Brückenmodul zugewandten Seite der Gelenkpfanne unterteilt sind. Bevorzugt sind die Abschnitte federelastisch ausgebildet, so dass ein Heraushebeln des Gelenkkopfes aus der Gelenkpfanne ermöglicht ist. Zudem führt die Unterteilung der Gelenkpfanne in mehrere Bereiche zu einer besseren Verformbarkeit der Gelenkpfanne bei dem Heraushebeln wie auch bei dem Wiedereinsetzen des Gelenkkopfes. Ferner wird durch die federelastischen Bereiche ein Bruch der Gelenkpfanne beim Aushebeln des Gelenkkopfes vermieden. Die Federelastizität der Abschnitte ist so gewählt, dass ein bestimmtes Moment oder eine bestimmte Kraft zu einem Aushebeln führt, wobei dieses Moment oder diese Kraft unkritisch für den Verbindungsbereich zwischen Endoprothese und Oberschenkelknochen ist.

Ein Heraushebeln des Gelenkkopfes aus der Gelenkpfanne und eine damit einhergehende Trennung der Exoprothese von der Endoprothese ist vorteilhaft, da bei einer Unfallsituation, trotz Verschwenken der Exoprothese gegenüber der Endoprothese nach Bruch der Scherelemente, eine zu hohe mechanische Belastung auf den Verbindungsbereich zwischen Endoprothese und Knochen wirken würde, sobald der Gelenkkopf seine maximale Verschwenkung innerhalb der Gelenkpfanne erreicht hat.

Vorzugsweise liegen die Abschnitte an ihrem dem Brückenmodul zugewandten Ende jeweils zumindest mit einem Vorsprung an dem Gelenkkopf an. Die Vorsprünge sorgen dafür, dass die unterteilten und federelastischen Bereiche der Gelenkpfanne unter Spannung an der Oberfläche des Gelenkkopfes anliegen. Mithin wird ein spielfreier Sitz des Gelenkkopfes in der Gelenkpfanne erzielt. Ebenfalls sorgen die Vorsprünge für eine erleichterte Montage des Gelenkkopfes innerhalb der Gelenkpfanne, da sie die Funktion von Rastelementen übernehmen.

Die Erfindung sowie das technische Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und Erkenntnissen aus der vorliegenden Beschreibung und/oder Figuren zu kombinieren. Insbesondere ist darauf hinzuweisen, dass die Figuren und insbesondere die dargestellten Größenverhältnisse nur schematisch sind. Gleiche Bezugszeichen bezeichnen gleiche Gegenstände, so dass gegebenenfalls Erläuterungen aus anderen Figuren ergänzend herangezogen werden können. Es zeigen:
- Fig. 1: eine schematische Schnittdarstellung eines erfindungsgemäßen Knieadapters, angeordnet an einer Endo- und Exoprothese;
- Fig. 2: eine schematische Darstellung der Unterseite des erfindungsgemäßen Knieadapters;
- Fig. 3: eine schematische Schnittdarstellung des erfindungsgemäßen Knieadapters mit überbeanspruchten Scherelementen;
- Fig. 4: eine schematische Schnittdarstellung des erfindungsgemäßen Knieadapters mit aus der Gelenkpfanne ausgerücktem Gelenkkopf und
- Fig. 5: eine schematische Seitenansicht des erfindungsgemäßen Knieadapters.

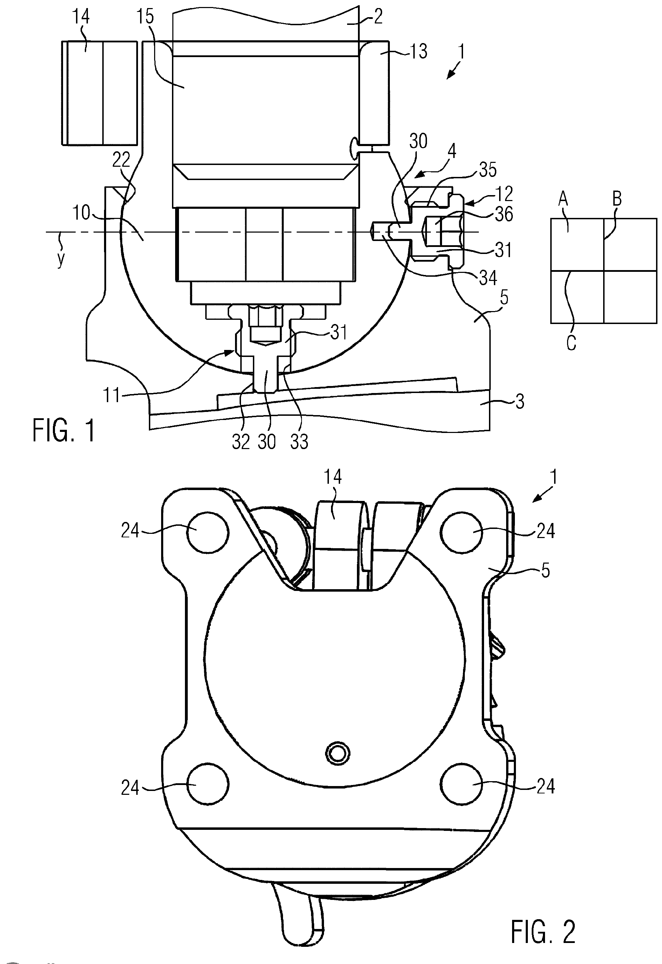

Fig. 1 zeigt eine schematische Darstellung eines erfindungsgemäßen Knieadapters 1. Der Knieadapter 1 ist dazu ausgebildet, eine als zumindest teilweisen Oberschenkelersatz ausgebildete und osseointegrierte Endoprothese 2 mit einer den Unterschenkel ersetzenden Exoprothese 3 zu verbinden. Der Knieadapter 1 umfasst ein Kugelgelenk 4, wobei das Kugelgelenk 4 einen teilweise kugelförmigen Gelenkkopf 10 und eine dazu komplementär geformte Gelenkpfanne 5 aufweist. Die Gelenkpfanne 5 und der Gelenkkopf 10 sind so zueinander ausgebildet, dass der Gelenkkopf 10 verschwenkbar innerhalb der Gelenkpfanne 5 gelagert ist. Der Gelenkkopf 10 weist einen Äquator Y auf einer Normalenebene zu seiner axialen Erstreckung auf, wobei die Gelenkpfanne 5 den Gelenkkopf 10 über diesen Äquator Y hinweg in Richtung des Brückenmoduls 13 zumindest teilweise umschließt. Durch diese Einnestung des Gelenkkopfes 10, bei Betrachtung des Knieadapters 1 in axialer Richtung über die 90° hinweg, wird ein Herausfallen des Gelenkkopfes 10 aus der Gelenkpfanne 5 verhindert.

Um die Endoprothese 2 mit der Exoprothese 3 zu verbinden, ist der Gelenkkopf 10 auf der von der Gelenkpfanne 5 abgewandten Seite mit einem hohlzylindrischen Brückenmodul 13 ausgebildet. Die Endoprothese 2 ist ihrerseits mit einem zu dem hohlzylindrischen Brückenmodul 13 komplementär ausgebildeten Verbindungsbereich 15 in Form eines zylindrischen Abschnittes 15 versehen, der in dem Brückenmodul 13 aufgenommen ist. Um den zylindrischen Abschnitt 15 der Endoprothese 2 ortsfest in dem hohlzylindrischen Brückenmodul 13 anzubinden, ist das Brückenmodul 13 mit einem Befestigungselement 14 versehen. Das Befestigungselement 14 ist als ringförmiger Schnellspanner umlaufend um den Bereich des Brückenmoduls 13 ausgebildet. Um eine Verbindung zwischen der Endoprothese 2 und dem Brückenmodul 13 zu schaffen, weist das Brückenmodul 13 eine Fuge 23 auf, die es ermöglicht, dass das Brückenmodul 13 durch das Befestigungselement 14 zusammengedrückt und so ein selbsthemmender Reibschluss, also Kraftschluss zwischen der Endoprothese 2 und dem Brückenmodul 13 hergestellt wird.

Darüber hinaus weist die Gelenkpfanne 5 auf der vom Gelenkkopf 10 abgewandten Seite eine flanschförmige Struktur auf, die dazu ausgebildet ist, eine Verbindung mit der Exoprothese 3 herzustellen. Um eine kraftschlüssige Verbindung der Endoprothese 2 mit der flanschförmigen Struktur der Gelenkpfanne 5 herzustellen, weist die flanschförmige Struktur der Gelenkpfanne 5, wie in Fig. 2 dargestellt, vier Gewindebohrungen 24 auf, die dazu ausgebildet sind, nicht dargestellte Muttern aufzunehmen, die die Exoprothese 3 mit der flanschförmigen Struktur der Gelenkpfanne 5 kraft- und formschlüssig miteinander verbinden.

Um den Gelenkkopf 10 innerhalb der Gelenkpfanne 5 festzulegen, sind ein Scherelement 11 und ein weiteres Scherelement 12 vorgesehen, die eine Bewegung des Gelenkkopfes 10 innerhalb der Gelenkpfanne 5 sperren. Dazu sind die beiden Scherelemente 11, 12 jeweils mit einem Ende in der Gelenkpfanne 5 und mit dem jeweils anderen Ende in dem Gelenkkopf 10 angeordnet. Die Scherelemente 11, 12 sind in axialer Richtung orthogonal zueinander angeordnet, wobei das Scherelement 11 eine rotatorische Bewegung des Gelenkkopfes 10 innerhalb der Gelenkpfanne 5 in Frontalebene A und in Sagittalebene B blockiert, wohingegen das weitere Scherelement 12 eine rotatorische Bewegung des Gelenkkopfes 10 innerhalb der Gelenkpfanne 5 in Transversalebene C und in Frontalebene A blockiert.

Die Scherelemente 11, 12 sind jeweils in zwei Abschnitte 30, 31 unterteilbar, nämlich in einen Gewindeabschnitt 31 und einen koaxial darauffolgenden, radial verjüngten zylindrischen Abschnitt 30. Das Scherelement 11 greift mit seinem verjüngten zylindrischen Abschnitt 30, ausgehend von der Innenseite des Gelenkkopfes 10, in eine erste Bohrung 32 innerhalb der Gelenkpfanne 5 ein, wobei der Gewindeabschnitt 31 in einer ersten Gewindebohrung 33 in dem Gelenkkopf 10 eingeschraubt ist. Mit anderen Worten ist das Scherelement 11 von innerhalb des Gelenkkopfes 10 in die erste Gewindebohrung 33 eingeschraubt. Die erste Gewindebohrung 33 ist koaxial zu dem hohlzylindrischen Brückenmodul 13 ausgebildet und erstreckt sich parallel zu der Schnittlinie zwischen der Frontalebene A und der Sagittalebene B.

Das weitere Scherelement 12 hingegen greift mit seinem verjüngten zylindrischen Abschnitt 30, ausgehend von der Außenseite der Gelenkpfanne 5, in eine zweite Bohrung 34 innerhalb des Gelenkkopfes 10 ein, wobei der Gewindeabschnitt 31 in einer zweiten Gewindebohrung 35 in der Gelenkpfanne 5 eingeschraubt ist. Mit anderen Worten ist das weitere Scherelement 12 von außerhalb der Gelenkpfanne 5 in die zweite Gewindebohrung 35 eingeschraubt und somit von außen her zugänglich. Die zweite Gewindebohrung 35 ist orthogonal zu der ersten Gewindebohrung 33 ausgebildet und erstreckt sich parallel zur Schnittlinie zwischen der Sagittalebene B und der Transversalebene C.

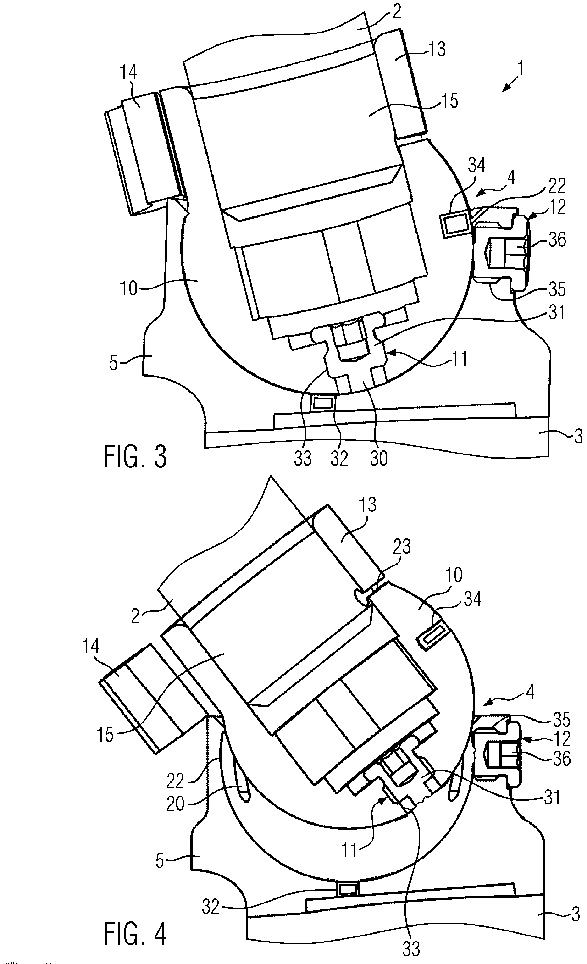

Gemäß Fig. 3 und 4 sind die zylindrischen Abschnitte 30 des Scherelements 11 und des Weiteren Scherelements 12 mit Sollbruchstellen ausgebildet. Wird ein bestimmtes Maß an Kraft oder Moment das auf sie wirkt überschritten, brechen die zylindrischen Abschnitte 30 und geben den Gelenkkopf 10 innerhalb der Gelenkpfanne 5 frei. Sodann kann der Gelenkkopf 10 innerhalb der Gelenkpfanne 5 frei verschwenken, wie in Fig. 3 dargestellt. Um einen Austausch der Scherelemente 11, 12 nach einer Überlastung und dem damit einhergehenden Bruch des zylindrischen Abschnitts 30 vom jeweiligen Gewindeabschnitt 31 zu ermöglichen, ist der jeweilige Gewindeabschnitt 31 mit einer Sacklochbohrung 36 versehen, wobei die Sacklochbohrungen 36 für die Aufnahme eines Sechskantschlüssels ausgebildet sind. Wie bereits dargelegt, kann das weitere Scherelement 12 nach einem Bruch von außerhalb des Kugelgelenks 4 leicht ersetzt werden. Um das Scherelement 11 nach einem Bruch auszutauschen muss zunächst die Endoprothese 2 von dem Knieadapter 1 beziehungsweise von dem Brückenmodul 13 getrennt werden, wonach das Scherelement 11 von innerhalb des Gelenkkopfes 10 ausgetauscht werden kann.

Die Scherelemente 11, 12 sind jeweils aus Edelstahl ausgebildet und weisen eine niedrige Duktilität auf. Eine niedrige Duktilität hat den Vorteil, dass bei einer genau festgelegten Belastung durch ein Moment oder eine Kraft der oder die zylindrischen Abschnitte 30 unverzüglich, also ohne eine vorhergehende plastische Verformung brechen. Alternativ bevorzugt können die Scherelemente 11, 12 auch aus anderem Material mit einer niedrigen Duktilität ausgebildet sein, beispielsweise Keramik. Da statistisch gesehen die höchsten Belastungen in und entgegen der Fortbewegungsrichtung des Prothesenträgers orthogonal, also in Richtung der Frontalebene A auf die Exoprothese 3 wirken, ist das Scherelement 11, genauer der zylindrische Abschnitt 30, stärker dimensioniert als das weitere Scherelement 12. Mithin weist das Scherelement 11 eine höhere Bruchfestigkeit gegenüber einer Scherbeanspruchung als das weitere Scherelement 12 auf. Belastungen quer zur Fortbewegungsrichtung des Nutzers, also in Richtung der Sagittalebene B, fallen hingegen schwächer aus, weswegen das weitere Scherelement 12 schwächer dimensioniert ist. Ebenfalls ist dessen Belastung im Normalbetrieb niedriger, so dass die mechanische Belastungsschwelle für den Bruch des Weiteren Scherelements 12 konstruktiv herabgesetzt werden kann.

Ferner ist die reine Torsionsbelastung sehr viel niedriger als die Biegebelastung bei einem Sturz oder Ähnlichem. Deshalb ist das Scherelement 12 kleiner dimensioniert als das Scherelement 11, da bei einer reinen Torsionsbelastung nur das Scherelement 12 abschert. Bei einer Biegebelastung werden je nach Achse entweder beide Scherelemente abscheren (große Belastung) oder bei reiner Biegung um Frontal/Transversalschnittachse nur das Scherelement 11.

Da die beiden Scherelemente 11, 12 verschiedenartig zueinander orientiert sind, ist es auch möglich, dass bei Überschreitung einer entsprechenden mechanischen Belastungsschwelle nur eines der Scherelement 11, 12 bricht. Dann wäre eine Schwenkbewegung des Gelenkkopfes 10 gegenüber der Gelenkpfanne 5 in axialer Richtung um das noch erhaltene Scherelement 11, 12 möglich.

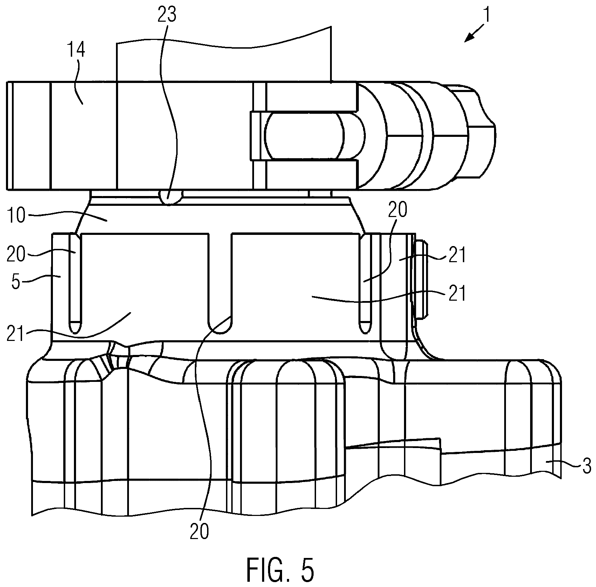

Wie in Fig. 4 dargestellt, ist der Knieadapter 1 dazu ausgebildet, dass sich der Gelenkkopf 10, nach einem Bruch der Scherelemente 11, 12, aus der Gelenkpfanne 5 aushebeln lässt. Sollte selbst nach dem Bruch der Scherelemente 11, 12 noch weiter eine Kraft oder ein Moment auf die Exoprothese 3 wirken, und somit potentiell eine Fraktur des Verbindungsbereichs zwischen dem Rest des Oberschenkelknochens und der Endoprothese 2 bestehen, kann die Exoprothese 3 von der Endoprothese 2 getrennt werden. Dazu ist ein den Gelenkkopf 10 radial umschließender Bereich der Gelenkpfanne 5 in mehrere Abschnitte 21 unterteilt (Fig. 5). Die Abschnitte 21 sind durch Aussparungen 20 in axialer Erstreckung der Gelenkpfanne 5 unterteilt, ausgehend von der dem Brückenmodul 13 zugewandten Seite der Gelenkpfanne 5. Um ein Heraushebeln des Gelenkkopfes 10 aus der Gelenkpfanne 5 zu ermöglichen, sind die Abschnitte 21 elastisch ausgebildet. Diese Elastizität ergibt sich vor allem durch die Formgebung der Abschnitte 21. Zudem führt die Unterteilung der Gelenkpfanne 5 in mehrere Abschnitte 21 zu einer besseren Verformbarkeit der Abschnitte 21 der Gelenkpfanne 5 beim Heraushebeln, wie auch wieder Einsetzen des Gelenkkopfes 10 in die Gelenkpfanne 5. Ferner wird durch die federelastischen Abschnitte 21 ein Bruch der Gelenkpfanne 5 beim Aushebeln des Gelenkkopfes 10 vermieden.

Die Federelastizität der Abschnitte 21 ist so gewählt, dass ein bestimmtes Moment oder eine bestimmte Kraft zu einem Aushebeln führt, wobei dieses Moment oder die Kraft unkritisch für den Verbindungsbereich zwischen Endoprothese 2 und Oberschenkelknochen sind. Um diese Kraft- beziehungsweise Momentenschwelle konstruktiv besser umsetzen zu können, liegen die Abschnitte 21 an ihrem dem Brückenmodul 13 zugewandten Ende jeweils mit einem Vorsprung 22 an dem Gelenkkopf 10 an. Dies sorgt ebenfalls für eine erleichterte Montage des Gelenkkopfes 10 innerhalb der Gelenkpfanne 5. Die Vorsprünge 22 sorgen ferner dafür, dass die unterteilten und federelastischen Abschnitte 21 der Gelenkpfanne 5 unter Spannung an der Oberfläche des Gelenkkopfes 10 anliegen. Mithin wird ein spielfreier Sitz des Gelenkkopfes 10 in der Gelenkpfanne 5 erzielt.

### Bezugszeichenliste

- 1: Knieadapter
- 2: Endoprothese
- 3: Exoprothese
- 4: Kugelgelenk
- 5: Gelenkpfanne

- 10: Gelenkkopf
- 11: Scherelement
- 12: weiteres Scherelement
- 13: Brückenmodul
- 14: Befestigungselement
- 15: Verbindungsbereich/zylindrischer Abschnitt

- 20: Aussparung
- 21: Abschnitt
- 22: Vorsprung
- 23: Fuge
- 24: Gewindebohrung

- 30: zylindrischer Abschnitt
- 31: Gewindeabschnitt
- 32: erste Bohrung
- 33: erste Gewindebohrung
- 34: zweite Bohrung
- 35: zweite Gewindebohrung
- 36: Sacklochbohrung

- A: Frontalebene
- B: Sagittalebene
- C: Transversalebene
- Y: Äquator

## Patentansprüche

1. Knieadapter (1) zur Verbindung einer Endoprothese (2) mit einer Exoprothese (3), **gekennzeichnet durch** ein Kugelgelenk (4), wobei das Kugelgelenk (4) einen teilweise kugelförmigen Gelenkkopf (10) und eine dazu komplementär geformte Gelenkpfanne (5) aufweist, wobei der Gelenkkopf (10) in der Gelenkpfanne (5) gelagert ist, wobei der Gelenkkopf (10) dazu ausgebildet ist, an einem von Endo- und Exoprothese (2,3) angeordnet zu werden, wobei die Gelenkpfanne (5) dazu ausgebildet ist, an der anderen von Endo- und Exoprothese (2,3) angeordnet zu werden, wobei der Gelenkkopf (10) durch zumindest ein Scherelement (11) in der Gelenkpfanne (5) festgelegt ist.

2. . Knieadapter (1) nach Anspruch 1, **gekennzeichnet durch** ein weiteres Scherelement (12), wobei das Scherelement (11) und das weitere Scherelement (12) orthogonal zueinander ausgerichtet sind, wobei das Scherelement (11) und das weitere Scherelement (12) jeweils mit einem Ende in der Gelenkpfanne (5) und mit dem jeweils anderen Ende in dem Gelenkkopf (10) angeordnet sind.

3. . Knieadapter (1) nach Anspruch 1 oder 2, **dadurch gekennzeichnet, dass** das Scherelement (11) ausgehend von der Innenseite des Gelenkkopfes (10) in die Gelenkpfanne (5) eingreift.

4. . Knieadapter (1) nach Anspruch 2 oder 3, **dadurch gekennzeichnet, dass** das Scherelement (11) und das weitere Scherelement (12) aus Edelstahl ausgebildet sind, wobei das Scherelement (11) eine höhere Bruchfestigkeit gegenüber einer Scherbeanspruchung als das weitere Scherelement (12) aufweist.

5. . Knieadapter (1) nach einem der vorhergehenden Ansprüche, **dadurch gekennzeichnet, dass** der Gelenkkopf (10) auf der von der Gelenkpfanne (5) abgewandten Seite ein Brückenmodul (13) aufweist, wobei das Brückenmodul (13) komplementär zu einem Verbindungsbereich (15) der Endoprothese (2) oder/und der Exoprothese (3) ausgebildet ist.

6. . Knieadapter (1) nach Anspruch 5, **dadurch gekennzeichnet, dass** an der der Gelenkpfanne (5) abgewandten Seite des Brückenmoduls (13) umfangsseitig ein Befestigungselement (14) angeordnet ist.

7. . Knieadapter (1) nach einem der vorhergehenden Ansprüche, **dadurch gekennzeichnet, dass** der Gelenkkopf (10) einen Äquator (Y) auf einer Normalenebene zu seiner axialen Erstreckung aufweist, wobei die Gelenkpfanne (5) den Gelenkkopf (10) über diesen Äquator (Y) hinweg in Richtung des Brückenmoduls (13) zumindest teilweise umschließt.

8. . Knieadapter (1) nach einem der vorhergehenden Ansprüche, **dadurch gekennzeichnet, dass** ein den Gelenkkopf (10) radial umschließender Bereich der Gelenkpfanne (5) in zumindest zwei Abschnitte (21) unterteilt ist, wobei die Abschnitte (21) durch Aussparungen (20) in axialer Erstreckung der Gelenkpfanne (5), ausgehend von der dem Brückenmodul (13) zugewandten Seite der Gelenkpfanne (5), unterteilt sind.

9. . Knieadapter (1) nach Anspruch 8, **dadurch gekennzeichnet, dass** die Abschnitte (21) an ihrem dem Brückenmodul (13) zugewandten Ende jeweils zumindest mit einem Vorsprung (22) an dem Gelenkkopf (10) anliegen.

10. . Knieadapter (1) nach Anspruch 8 oder 9, **dadurch gekennzeichnet, dass** die Abschnitte (21) federelastisch ausgebildet sind.
